# EUROPÄISCHE PATENTSCHRIFT

(11) **EP 1 247 447 B1**
(45) Veröffentlichungstag und Bekanntmachung des Hinweises auf die Patenterteilung: **15.09.2004**
(21) Anmeldenummer: 01107796.3
(22) Anmeldetag: 05.04.2001
(51) Int. Cl.: A01M 1/20, A61L 9/03

(54) **Vorrichtung zum Verdampfen von flüchtigen Substanzen, insbesondere von Insektiziden und/oder Duftstoffen**
Device for vaporising fluids, particularly insecticides and/or perfumes
Dispositif de vaporisation de substances liquides, notamment insecticides et/ou parfums

(43) Veröffentlichungstag der Anmeldung: 09.10.2002
(73) Patentinhaber: C.T.R., Consultoria, Técnica e Representaçoes Lda, 2715-251 Almargem do Bispo (PT)
(72) Erfinder: Vieira, Pedro Queiroz, 2775 - 178 Parede (PT)
(74) Vertreter: Liebl, Thomas, Dipl.-Ing.

(56) Entgegenhaltungen:
- EP-A- 0 911 041
- EP-A- 1 055 430
- WO-A-01/05442

## Beschreibung

Die Erfindung betrifft eine Vorrichtung zum Verdampfen von flüchtigen Substanzen, insbesondere von Insektiziden und/oder Duftstoffen, nach dem Oberbegriff des Anspruchs 1.

Derartige Vorrichtungen zum Verdampfen sind allgemein bekannt. So sind beispielsweise Verdampfungsvorrichtungen bekannt, bei denen ein in eine Verdampfungsvorrichtung eingesetztes und mit einem Wirkstoff imprägniertes Plättchen erhitzt wird, um den Wirkstoff zu verdampfen. Weiter ist es auch bekannt, in ein Gehause einer Verdampfungsvorrichtung einen eine flüchtige Substanz enthaltenden Behälter einzusetzen. Dieser Behälter umfasst einen Docht, der die zu verdampfende Substanz mittels der Kapillarwirkung aus dem Behälter fördert, wobei das aus dem Behälter ragende Dochtende benachbart zu einem Heizelement, wie z. B. einem Keramikblock, angeordnet ist, so dass die Substanz durch die vom Keramikblock abgestrahlte Wärme verdampft und über Lüftungsschlitze im Gehäuse aus diesem in die Umgebung entweichen kann.

So ist beispielsweise aus der gattungsgemäßen EP 0 943 344 A1 eine Vorrichtung zum Verdampfen von flüchtigen Substanzen, insbesondere von Insektiziden und/oder Duftstoffen, bekannt, die ein Gehäuse mit einer darin angeordneten Heizeinrichtung aufweist, die wiederum einen Heizblock aus Keramik umfasst. Dieser Heizblock weist zu dessen Erwärmung ein Heizelement auf. Ferner umfasst diese Verdampfungsvorrichtung einen mit dem Gehäuse verbindbaren Behälter für eine zu verdampfende Substanz, wobei in den Behälter ein Docht einsetzbar ist, der bei mit dem Gehäuse verbundenen Behälter zur Verdampfung der sich im Behälter befindlichen Substanz mit einem aus dem Behälter ragenden Dochtende in eine Dochtaussparung des Heizblocks ragt.

Konkret besteht die Verdampfungsvorrichtung hier aus einem einen Anschlussstecker aufweisenden Steckerteil. Das Steckerteil weist ein Gewinde auf und wird in das Gehäuse eingesetzt, in das auch der Behälter für die zu verdampfende Substanz eingesetzt wird. Am Gehäuse sind Stiftausnehmungen vorgesehen, in die die Raststifte so eingesetzt werden, dass diese in das Gewinde des Steckerteils eingreifen. Dadurch kann der Abstand der mit dem Steckerteil verbundenen Heizeinrichtung relativ zu einem aus dem Behälter ragenden Dochtende durch Verdrehen des Steckerteils verändert werden. In einer Ausgestaltung hierzu kann das Steckerteil zudem exzentrisch im Gehäuse gelagert sein, so dass auch hierüber der Relativabstand zwischen dem Dochtehde und der Heizeinrichtung je nach dem gewünschten Verdampfungsgrad verändert werden kann. Mit einer derartigen Verdampfungsvorrichtung, in die ein Behälter einsetzbar ist, kann jeweils eine einzige bestimmte Substanz verdampft werden, z. B. ein Insektizid zur Insektenvernichtung oder Insektenvertreibung, ein Duftstoff zur Luftverbesserung und/oder zur Verwendung im Rahmen einer Aromatherapie. Insbesondere bei der Verwendung im Rahmen einer Aromatherapie ist es oftmals erforderlich, zwei oder ggf. auch mehr Duftstoffe miteinander zu verdampfen, wofür je nach der Anzahl der zu vermengenden und zu verdampfenden Duftstoffe eine entsprechende Mehrzahl derartiger Verdampfungsvorrichtungen benötigt wird. Ein Einsatz mehrerer Verdampfungsvorrichtungen kann z.B. auch in Verbindung mit der Verdampfung von z.B. zwei unterschiedlichen Insektiziden, die auf bestimmte Insektenarten abgestimmt sind, erforderlich sein.

Weiter nachteilig ist, dass ein derartiger Aufbau einer Verdampfungsvorrichtung, insbesondere im Hinblick auf die Einstellbarkeit des Verdampfungsgrades, relativ aufwendig und kompliziert ist, so dass der Herstellaufwand und damit auch die Verdampfungsvorrichtungen selbst insgesamt relativ teuer sind.

Ein ähnlicher Aufbau mit den eben genannten Nachteilen ist auch aus der WO 98/58692, WO 98/19526 und der EP 0 962 132 A1 bekannt, denen ebenso wie der gattungsgemäßen EP 0 943 344 A1 die Aufgabe zugrunde liegt, durch Relativverstellung des Dochtes zum Heizblock den Verdampfungsgrad zu ändern.

Zudem ist all diesen bekannten Verdampfungsvorrichtungen gemeinsam, dass sie insgesamt relativ großbauend ausgebildet sind und somit optisch weniger ansprechend sind, was den optischen Gesamteindruck, z. B. in einem Wohnraum, beeinträchtigen kann. Insbesondere wird ein derartiger großbauender Aufbau auch durch die Vielzahl von Bauteilen bewirkt, mit denen eine Einstellung des Verdampfungsgrads erzielt werden soll.

Es ist daher Aufgabe der Erfindung, eine alternative Vorrichtung zum Verdampfen von flüchtigen Substanzen, insbesondere von Insektiziden und/oder Duftstoffen zu schaffen, deren Einsatzmöglichkeiten, insbesondere auch im Rahmen einer Aromatherapie, wesentlich erhöht sind und die zudem ohne großen Bauteilaufwand relativ einfach und preiswert herstellbar ist.

Diese Aufgabe wird gelöst mit den Merkmalen des Anspruch 1.

Gemäß Anspruch 1 ist im Heizblock wenigstens eine weitere Dochtaussparung ausgebildet, wobei jeder weiteren Dochtaussparung jeweils ein weiterer Behälter mit darin einsetzbarem Docht so zugeordnet ist, dass ein Dochtende des Dochtes des weiteren Behälters zur Verdampfung der sich in diesem weiteren Behälter befindlichen Substanz in die weitere Dochtaussparung hineinragt.

Mit einem derartigen Aufbau wird vorteilhaft erreicht, dass gleichzeitig zwei oder auch mehr flüchtige Substanzen mit einer einzigen Vorrichtung verdampft werden können. Je nach Verwendungszweck können dies z. B. insbesondere im Rahmen einer Aromatherapie oder aber auch bei einer Raumluftverbesserung zwei oder mehr verschiedene Duftstoffe sein, die dann mit ein und derselben Vorrichtung gleichzeitig verdampft werden. Ebenso können mit ein und derselben Vorrichtung auch unterschiedliche Insektizide, die beispielsweise auf unterschiedliche Insektenarten abgestimmt sind, gleichzeitig verdampft werden. Hierbei ist lediglich sicherzustellen, dass der Heizblock durch das Heizelement so erwärmt wird, dass sich an diesem eine Verdampfungstemperatur im Bereich der Dochtaussparungen einstellt, die die Verdampfung aller zu verdampfenden Substanzen ermöglicht.

Somit können mit dem vorliegenden Erfindungsgegenstand vorteilhaft auf einfache und preiswerte Weise mit einer einzigen Vorrichtung verschiedene Insektizide oder Duftstoffkombinationen verdampft werden. Dadurch ist es insbesondere in Verbindung mit Duftstoffgemischen, z.B. im Rahmen einer Aromatherapie, oder im Falle des Einsatzes von zwei oder mehr auf bestimmte Insektenarten genau abgestimmten Insektiziden nicht mehr erforderlich, mehrere derartiger Vorrichtungen bereitzustellen, um eine entsprechende Vermischung von unterschiedlichen flüchtigen Substanzen zu erzielen.

Sollte nur die Verdampfung einer einzigen Substanz gewünscht sein, ist dies ebenfalls nach wie vor möglich, da dann z. B. lediglich ein Behälter in das Gehäuse eingesetzt wird.

Des weiteren können mit der erfindungsgemäßen Vorrichtung auch zwei in unterschiedlichen Behältern aufgenommene gleiche Substanzen verdampft werden, so dass eine schnellere Verdampfung einer größeren Menge an flüchtigen Substanzen mit einer einzigen Vorrichtung erfolgen kann, was beispielsweise insbesondere bei großen Räumen erforderlich sein kann.

Insgesamt ergeben sich hierdurch vielseitige Einsatz- und Verwendungsmöglichkeiten mittels einer einzigen Vorrichtung zum Verdampfen von flüchtigen Substanzen in Verbindung mit den jeweils zu verdampfenden Substanzen, wobei eine einfache Anpassung und Umstellung auf die jeweiligen individuellen Einsatzfälle schnell möglich ist, so dass diese Vorrichtung insgesamt sehr vielfältig einsetzbar ist.

Grundsätzlich können im Heizblock mehr als zwei Dochtaussparungen mit entsprechend zugeordneten Dochten von Behältern vorgesehen sein. Um jedoch nach wie vor einen besonders kompakten Aufbau der Vorrichtung zum Verdampfen von flüchtigen Substanzen zu gewährleisten, ist es gemäß einer besonders bevorzugten Ausführungsform nach Anspruch 2 vorgesehen, dass das Gehäuse mit zwei Behältern verbindbar ist, die jeweils einen Docht aufweisen. Dazu sind dann im Heizblock zwei Dochtaussparungen ausgebildet, in die jeweils ein Dochtende eines jedem Behälter zugeordneten Dochtes ragt.

Grundsätzlich können gemäß Anspruch 3 die beiden Behälter jeweils separate Behältnisse sein, die jeweils einen einzigen Docht aufweisen. Alternativ dazu ist jedoch ebenfalls nach Anspruch 3 auch ein einziges Behältnis mit zwei voneinander abgetrennten Kammern als Behälter möglich, wobei in den beiden als Kammern ausgebildeten Behältern z. B. unterschiedliche zu verdampfende Substanzen aufgenommen sein können, in die jeweils ein Docht ragt. Insbesondere in letzterem Fall ergibt sich ein besonders kompakter Aufbau der Verdampfungsvorrichtung im praktischen Einsatz.

Ein kleinbauendes und gut geeignetes Heizelement mit einer guten Heizleistung wird gemäß Anspruch 4 durch ein elektrisches Widerstandselement gebildet, das im Heizblock aufgenommen ist.

Eine besonders vorteilhafte und einfache sowie funktionssichere Zuordnung des Dochtendes zum Heizblock und damit zur Heizungseinrichtung ist gemäß Anspruch 5 möglich, wenn die Dochtaussparungen als Durchgangsloch oder als am Heizblock randseitig durchgehende Einbuchtungen ausgebildet sind. Dies trägt dazu bei eine besonders effektive Verdampfung zu gewährleisten.

Vorteilhaft ist das Heizelement bezogen auf die Draufsicht auf den Heizblock in etwa in einem mittleren Bereich zwischen zwei Dochtaussparungen angeordnet. Eine besonders gute und effektive Verdampfung ergibt sich hierbei gemäß Anspruch 6 dann, wenn der Heizblock in einer Draufsicht eine rechteckige oder in etwa ovale Form aufweist und das Heizelement bezogen auf die Draufsicht in etwa in einem mittleren Bereich dieses Heizblocks angeordnet ist, so dass jeweils eine Dochtaussparung zu beiden Seiten des Heizelements liegt. Dadurch ergibt sich am Heizblock eine gute Wärmeleitung in Richtung zu den beiden Dochtaussparungen hin, die eine optimale Verdampfung von den beiden zu verdampfenden flüchtigen Substanzen ermöglicht, da im Bereich jeder Dochtaussparung eine gleich schnelle und gleich hohe Verdampfungstemperatur einstellbar ist. Die Heizblocktemperatur soll dabei wenigstens so hoch sein wie die Verdampfungstemperatur derjenigen flüchtigen Substanz, die die höchste Verdampfungstemperatur aufweist. Allerdings ist es auch ausreichend, den Heizblock so zu gestalten, dass in den Dochtaussparungsbereichen die gewünschte jeweilige Verdampfungstemperatur vorliegt, was auch bei unterschiedlichen Temperaturen in unterschiedlichen Heizblockbereichen der Fall sein kann

Eine besonders vorteilhafte gleichmäßige und effektive Verdampfung mit einer guten Vermischung von zwei unterschiedlichen zu verdampfenden Substanzen ergibt sich mit den Merkmalen des Anspruchs 7, wenn die Dochtaussparungen jeweils gleich mit einem gleichen Abstand zum Heizelement für eine vorzugsweise symmetrische Anordnung der Dochtaussparungen bezüglich des Heizelements ausgebildet sind.

Gemäß einer besonders bevorzugten Weiterbildung der Erfindung können gemäß Anspruch 8 wenigstens zwei Heizelemente am Heizblock vorgesehen sein, wobei jedes Heizelement wenigstens einer Dochtaussparung zugeordnet ist und wobei jede Dochtaussparung sowie jedes einer Dochtaussparung zugeordnete Heizelement jeweils eine Heizeinheit bilden. Die Heizelemente sind dabei mit einer Schalt- und/oder Steuereinrichtung gekoppelt, über die die Heizelemente zusammen deaktivierbar sowie ggf. aktivierbar sind und über die die Heizelemente einzeln aktivierbar und deaktivierbar sind. Mit einem derartigen Aufbau ergibt sich eine noch höhere Flexibilität und eine noch bessere Funktionsintegration dadurch, dass je nach der Anzahl der Heizelemente und der entsprechend zugeordneten Dochtaussparungen eine Vielzahl von einzelnen Heizeinheiten jeweils bestehend aus Heizelement und Dochtaussparung in einer einzigen Vorrichtung integriert werden können. Über die Schalt- und Steuereinrichtung können diese einzelnen Heizeinheiten jeweils zusammen oder einzeln aktiviert und deaktiviert werden, so dass eine individuelle Zu- und Abschaltung je nach den gerade gegebenen Verdampfungssituationen und - wünschen möglich ist.

Grundsätzlich kann jeweils ein Heizelement einer Dochtaussparung zugeordnet sein. Jedoch ist es auch möglich, dass einer Dochtaussparung mehr als ein Heizelement, z.B. mit unterschiedlichen Heizleistungen, zugeordnet ist, so dass ein und demselben Dochtaussparungsbereich je nach gerade aktiviertem zugeordneten Heizelement unterschiedliche Verdampfungsleistungen, z.B. schnell oder langsam, zuordenbar sind. Ggf. können auch alle zugeordneten Heizelemente gemeinsam aktiviert sein oder aber auch bloß einzelne von mehreren, z.B. paarweise, aktiviert sein. Ebenso ist es aber auch möglich, dass einem Heizelement mehrere Dochtaussparungen zugeordnet sind, so dass ggf. über dieses Heizelement mehrere Dochtaussparungsbereiche auf Verdampfungstemperatur erwärmt werden können. Insgesamt kann somit die Verdampfungsleistung mit einem derartigen Aufbau auf besonders einfache Weise gut auf die gerade zu verdampfende Substanz abgestimmt werden, wobei aufgrund der Funktionsintegration an einem einzigen Bauteil ein sehr vielfältiger multifunktioneller Einsatz desselben möglich ist.

Für den Fall, dass die einzelnen Heizelemente verschiedene Temperaturen und damit Verdampfungsleistungen , z.B. langsam oder schnell, erzeugen sollen, können somit Heizelemente mit unterschiedlichen Heizleistungen eingesetzt werden. Dies stellt eine einfache und preiswerte Alternative zu den aus dem Stand der Technik bekannten Verdampfungsvorrichtungen dar, bei denen der Verdampfungsgrad lediglich in baulich aufwendiger und komplizierter Weise mit mechanischen Hilfsmitteln durch Relativabstandseinstellung verstellbar ist. Diese mechanischen Hilfsmittel, wie z.B. die eingangs in Verbindung mit der EP 0943 344 A1 erwähnten Gewinde können sich ggf. zusetzen, so dass die Raststifte dort nicht mehr in der gewünschten Weise eingreifen können, was die Funktion beeinträchtigen kann.

Alternativ ist jedoch auch der Einsatz von gleichen Heizelementen, d.h. von eine gleiche Heizleistung erzeugenden Heizelementen möglich, wenn z.B. unterschiedliche Substanzen verdampft werden sollen, die in etwa eine gleiche Verdampfungstemperatur aufweisen.

Gemäß einer nach Anspruch 9 besonders bevorzugten konkreten Ausführungsform, bei der zwei Heizelemente vorgesehen sind, die jeweils durch ein elektrisches Widerstandselement gebildet sind und die beabstandet voneinander am Heizblock angeordnet sind, ist vorgesehen, dass die beiden Heizelemente deaktiviert sind oder dass lediglich eines der Heizelemente aktiviert ist. Im letzteren Falle wird lediglich der diesem Heizelement zugeordnete Heizblockbereich am Durchgangsloch auf die dort erforderliche Verdampfungstemperatur in Abhängigkeit von der über dieses Durchgangsloch zu verdampfenden flüchtigen Substanz erwärmt. Alternativ dazu können jedoch auch beide Heizelemente gleichzeitig aktiviert sein, um beide flüchtigen Substanzen gleichzeitig zu verdampfen. Eine derartige Verdampfungsvorrichtung ist somit in besonders vielfältiger Weise einsetzbar.

Bevorzugt sind die elektrischen Widerstandselemente nach Anspruch 10 in etwa stabförmig ausgebildet und in etwa parallel zueinander ausgerichtet. Dadurch ergibt sich eine besonders gute Erwärmung des Heizblocks, insbesondere eines Keramikheizblocks, wobei diese stabförmigen Widerstandselemente zugleich lediglich einen geringen Platzbedarf aufweisen.

Grundsätzlich gibt es verschiedene Möglichkeiten die beiden Dochtaussparungen sowie die entsprechend zugeordneten beiden Heizelemente am Heizblock anzuordnen. Um jedoch die einzelnen Heizeinheiten bestehend aus Heizelement und entsprechend zugeordneter Dochtaussparung separat voneinander aktivieren und deaktivieren zu können, ohne dass es durch die eine Heizeinneit auch zu einer Aufheizung des Bereichs der anderen Heizeinheit, insbesondere des Durchgangslochbereichs, auf die dortige Verdampfungstemperatur kommt, wird gemäß Anspruch 11 vorgeschlagen, dass zwei Dochtaussparungen beabstandet voneinander sowie bezogen auf eine Draufsicht auf den Heizblock in einem mittleren Bereich zwischen zwei vorzugsweise im randseitigen Bereich des Heizblocks angeordneten Heizelementen angeordnet sind. Mit einer derartigen konkreten Anordnung der Heizelemente im ausreichenden Abstand von der anderen Heizeinheit und insbesondere von der anderen Dochtaussparung wird auf einfache Weise erreicht, dass keine Wärmeleitung in den Bereich der anderen Heizeinheit bzw. der anderen Dochtaussparung dergestalt erfolgen kann, dass der dortige Bereich auf eine Verdampfungstemperatur einer dort eventuell vorhandenen und nicht zu Verdampfen gewünschten flüchtigen Substanz erwärmt wird.

Diese thermische Entkopplung der unterschiedlichen Heizeinheiten wird mit den Maßnahmen des Anspruchs 12 noch erheblich verstärkt, wenn zur zumindest teilweisen thermischen Entkopplung der beiden durch je ein Heizelement und eine zugeordnete Dochtersparung gebildeten Heizeinheiten in einem Bereich zwischen den beiden Dochtaussparungen wenigstens ein Trennmittel vorgesehen ist. Dieses Trennmittel ist gemäß Anspruch 13 bevorzugt durch einen wenigstens im Bereich zwischen den beiden Dochtaussparungen durch den Heizblock durchgehenden Luftspalt gebildet. Eine derartige thermische Entkopplung mittels Trennmittel wie beispielsweise des Luftspaltes ist grundsätzlich auch bei Verdampfungsvorrichtungen möglich, bei denen mehr als zwei Heizeinheiten, bestehend aus z.B. einem Heizelement und einem Durchgangsloch, vorgesehen sind.

Ein weiterer wesentlicher Vorteil in Verbindung mit einer wenigstens zwei Heizelemente aufweisenden Vorrichtung ist, dass die Heizelemente gemäß Anspruch 14 jeweils eine unterschiedliche Heizleistung für unterschiedliche zu verdampfende Substanzen bereitstellen können. So können z.B. im Falle eines etektrischen Widerstandselementes als Heizelement diese unterschiedliche Widerstandswerte aufweisen. Damit ist eine gute Abstimmung der Verdampfungstemperatur auf die jeweils zu verdampfenden Substanzen möglich, was sich positiv auf den Verdampfungsgrad insgesamt auswirkt. Insbesondere können dadurch unterschiedliche Verdampfungsleistungen, z.B. langsam oder schnell, einfachst eingestellt werden. Grundsätzlich können jedoch auch zwei gleiche Widerstandselemente vorgesehen sein, z.B. für eine Verdampfung von in etwa eine gleiche Verdampfungstemperatur aufweisenden Substanzen.

Je nach Einsatzzweck der Vorrichtung gibt es nach Anspruch 15 verschiedene Möglichkeiten die Schalt- und/oder Steuereinrichtung auszubilden. Diese kann insbesondere in Verbindung mit einem kompakten, kleinbauenden Gerät direkt an diesem, z.B. am Gehäuse, integriert sein und als Handschalter ausgebildet sein. Alternativ dazu ist es aber auch möglich, die Schalt- und/oder Steuereinrichtung durch einen programmierbaren Mikroprozessor auszubilden, der entsprechend mit der Vorrichtung bzw. dem Gehäuse gekoppelt ist.

Gemäß Anspruch 16 ist hier das Heizelement über elektrische Leitungen mit einem am Gehäuse angeordneten Anschlussstecker gekoppelt. Damit ist ein einfacher und kompakter Aufbau der Vorrichtung insgesamt möglich, wobei zudem ein beliebiger Einsatz in unterschiedlichen Räumen bzw. Gebäuden etc. möglich ist.

Grundsätzlich kann das elektrische Widerstandselement durch jedes bekannte Widerstandselement gebildet sein, z. B. durch PTC-Widerstände. In einer besonders bevorzugten Ausgestaltung nach Anspruch 17 weist jedes elektrische Widerstandselement zur Ausbildung einer Heizeinrichtung mit geringen Abmessung und damit einer insgesamt miniaturisierten Vorrichtung zum Verdampfen von flüchtigen Substanzen einen stabförmigen Widerstandkörper auf, der wenigstens bereichsweise mit einer Widerstandsschicht beschichtet ist, die zur Einstellung eines bestimmten Widerstandswertes entsprechend einer auf die Zusammensetzung der jeweils zu verdampfenden Substanz abgestimmten Verdampfungstemperatur bereichsweise eingeschnitten und/oder bereichsweise eingeschliffen ist.

Vorteilhaft kann ein derartiges Widerstandselement für Heizeinrichtungen von Verdampfungsvorrichtungen relativ kleinbauend ausgebildet werden, so dass auch der Heizblock und die Heizeinrichtung und damit auch das die Heizeinrichtung aufnehmende Gehäuse insgesamt relativ kleinbauend ausgebildet werden können. Dadurch können beim gleichzeitigen Einsatz eines oder auch von zweien oder mehreren entsprechend angepassten kleinvolumigen Behältern in das Gehäuse Verdampfungsvorrichtungen mit geringen Abmessungen, d. h. eine miniaturisierte Verdampfungsvorrichtung geschaffen werden. Eine derartige miniaturisierte Verdampfungsvorrichtung ist aufgrund des reduzierten Material und Bauteilaufwands zudem auch relativ einfach und damit preiswert herstellbar, z. B. als einmal benutzbarer Wegwerfartikel.

Ein weiterer besonderer Vorteil einer derartigen Verdampfungseinrichtung ist, dass die Verdampfungstemperatur mit einem derartigen Widerstandselement, bei der die Widerstandsschicht zur Einstellung eines bestimmten Widerstandswertes bereichsweise eingeschnitten und/oder bereichsweise eingeschliffen ist, optimal auf die Zusammensetzung der jeweils zu verdampfenden Substanz abgestimmt werden kann. Dadurch kann z. B. auch vorteilhaft die Gefahr einer Entflammbarkeit der Vorrichtung insgesamt durch bestimmte Bestandteile reduziert und zudem auch eine ggf. negative Auswirkung auf den Verdampfungsgrad vermieden werden.

Grundsätzlich gibt es verschiedene Möglichkeiten die Widerstandsschicht zur Einstellung eines bestimmten Widerstandswertes einzuschneiden bzw. einzuschleifen. Gemäß einer bevorzugten Ausführungsform nach Anspruch 18 wird die Widerstandsschicht um den stabförmigen, vorzugsweise zylindrischen Widerstandskörper herum spiralförmig eingeschnitten, vorzugsweise durch Laserspiralschneiden. Mit einem derartigen spiralförmigen Einschnitt kann der Widerstandswert für eine optimale Verdampfungsleistung auf besonders einfache Weise sehr genau eingestellt werden. Die Widerstandsschicht kann grundsätzlich ebenfalls aus unterschiedlichen Materialien hergestellt sein, so z. B in Form einer Edelmetallschicht. Bevorzugt ist die Widerstandsschicht jedoch als Metalloxidschicht ausgebildet, vorzugsweise als Nickel-Chromlegierungsschicht, die vorteilhaft thermochemisch aufgebrannt, z. B. als Dünnschicht im Vakuum aufgedampft bzw. gesputtert, wird. Nach der Aufbringung der Widerstandsschicht wird diese vorzugsweise einem thermischen Verfahren unterworfen, um die Widerstandsschicht zu stabilisieren. Der Widerstandskörper kann dabei aus Keramik hergestellt sein, vorzugsweise mit einem hohen Gehalt an AL₂O₃ (Aluminiumoxid), wodurch eine besonders gute Wärmeleitfähigkeit des Widerstandskörpers und damit des Widerstandselementes insgesamt erreichbar ist. Der Gehalt an AL₂O₃ ist abhängig von den jeweils konkret gegebenen Einbauverhältnissen, z. B. dem verwendeten Gehäusematerial, Dochtmaterial etc.

Auf die Enden des beschichteten stabförmigen Widerstandskörpers sind Metallkappen aufsetzbar, die vorzugsweise aufgepresst sind. An diesen Kappen ist jeweils eine elektrische Leitung angebracht, vorzugsweise angeschweißt, die jeweils mit dem Anschlussstecker gekoppelt sind. Vorzugsweise werden als elektrische Leitungen für eine gute elektrische Leitung Kupferdrähte verwendet. In derartigen Metallkappen wird zudem ein guter elektrischer Kontakt zu der Widerstandsschicht auf einfache und funktionssichere Weise hergestellt.

Grundsätzlich gibt es verschiedene Möglichkeiten, das stabförmige Widerstandselement am Heizblock anzuordnen. In einer besonders bevorzugten Ausführungsform nach Anspruch 19 ist vorgesehen, dass das stabförmige Widerstandselement in eine Aussparung des Heizblocks einbringbar ist, wobei das Widerstandselement dort mit einem eine hohe Wärmeleitfähigkeit aufweisenden Material vergossen ist, um das Widerstandselement im Heizblock sicher zu fixieren. Das eine hohe Wärmeleitfähigkeit aufweisende Material ist vorzugsweise ein flammenbeständiger Isolationszement. Weiter ist an gegenüberliegenden Aussparungsenden der Aussparung zu beiden Seiten des Widerstandselementes vorzugsweise jeweils ein Schlitz ausgebildet, durch die die elektrischen Leitungen aus dem Heizblock zu dem Anschlussstecker hin herausgeführt sind. Mit einem derartigen Aufbau kann das Widerstandselement im Rahmen der Montage auf einfache Weise in die Aussparung eingesetzt werden, z. B. auch mit einem Klemmschluss, so dass das Widerstandselement beim Vergießen nicht mehr verrutschen kann. Weiter können die elektrischen Leitungen mit einem derartigen Aufbau auf einfache Weise auch in Richtung zu dem Anschlussstecker hin gebogen werden. Die elektrischen Leitungen können dabei auf herkömmliche Weise isoliert sein.

Eine besonders einfache und schnelle Montage der Heizeinrichtung im Gehäuse ist gemäß Anspruch 20 dann möglich, wenn das Gehäuse wenigstens zweiteilig aus einer Oberschale und einer Unterschale aufgebaut ist. Die Oberschale und die Unterschale können beispielsweise durch Rast- und/oder Clipelemente miteinander verbunden werden. In der Unterschale sind vorzugsweise Verbindungsmittel zur Verbindung des Behälters mit dem Gehäuse ausgebildet, z. B. Rastelemente. Wenigstens eine der beiden Schalen weist dabei auch wenigstens einen Lüftungsschlitz zum Entweichen der verdampften Substanz in die Umgebung auf. Die Lüftungsschlitze sind dabei vorzugsweise im Bereich oberhalb des Dochtendes in der Oberschale ausgebildet. Ein derartiges zweiteiliges Gehäuse ist auf besonders einfache und preiswerte Weise herstellbar.

Es zeigen:
- Fig. 1: eine schematische, vergrößerte Draufsicht auf eine Heizeinrichtung mit einem Heizblock mit zwei Dochtaussparungen und einem zentralen Heizelement,
- Fig. 2: eine schematische Seitenansicht der Darstellung gemäß Figur 1,
- Fig. 3: eine schematische, perspektivische Darstellung der Heizeinrichtung der Figuren 1 und 2,
- Fig. 4: eine schematische, perspektivische Darstellung eines zwei Kammern aufweisenden Behältnisses mit jeder Kammer zugeordnetem Docht sowie einer Heizeinrichtung entsprechend den Figuren 1 bis 3,
- Fig. 5: eine schematische, perspektivische Darstellung entsprechend Figur 4 mit montierter Heizeinrichtung und montierter Unterschale eines Gehäuses,
- Fig. 6: eine schematische, perspektivische Darstellung entsprechend der Figuren 4 und 5 mit fertig montiertem Gehäuse,
- Fig. 7: eine schematische Draufsicht auf ein durch ein elektrisches Widerstandselement gebildetes Heizelement,
- Fig. 8: ein schematischer Querschnitt durch den Heizblock entlang der Linie A-A der Fig. 1,
- Fig. 9: eine schematische, perspektivische Darstellung einer weiteren Ausführungsform einer Heizeinrichtung mit Heizblock mit zwei Durchgangslöchern und zwei Heizelementen,
- Fig. 10: eine schematische Draufsicht auf den Heizungsblock der Fig. 9,
- Fig. 11: ein schematischer Querschnitt entlang der Linie B-B der Fig. 10 und
- Fig. 12-14: den schrittweisen Zusammenbau einer Verdampfungsvorrichtung mit einer Heizeinrichtung entsprechend den Figuren 9 bis 11.

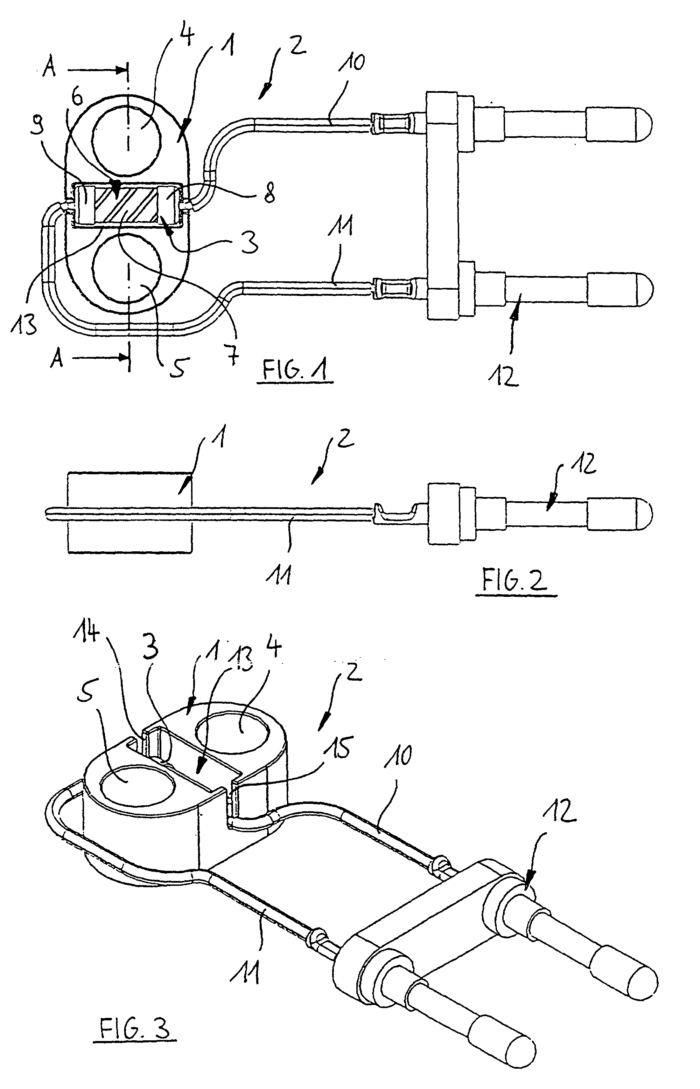

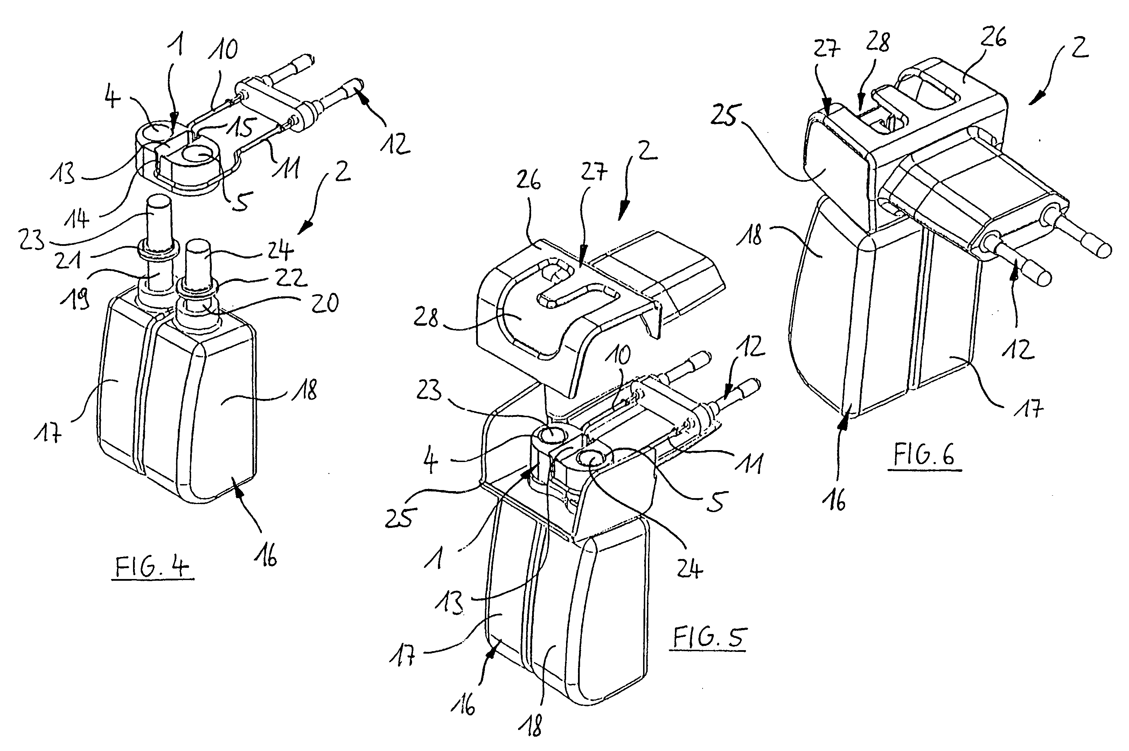

In den Figuren 1 bis 3 sind vergrößerte, unterschiedliche Ansichten einer Heizeinrichtung mit einem Heizblock 1 einer Verdampfungsvorrichtung 2 zum Verdampfen von flüchtigen Substanzen, insbesondere von Insektiziden und/oder Duftstoffen, dargestellt.

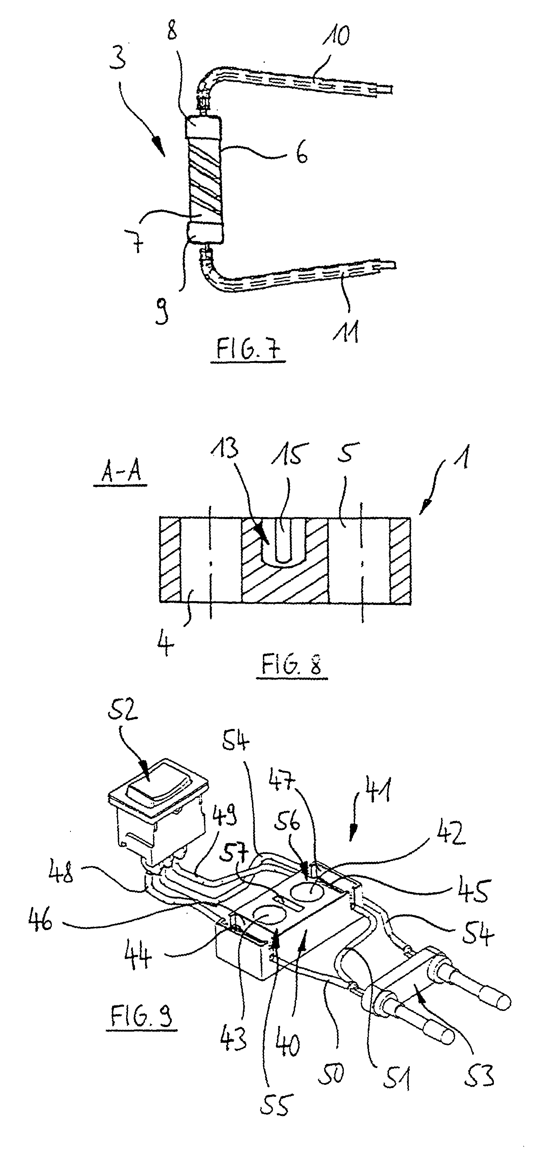

Wie dies insbesondere aus der Draufsicht der Fig. 1 ersichtlich ist, weist der vorzugsweise aus Keramik hergestellte Heizblock 1 in etwa eine ovale Form auf, wobei ein elektrisches Widerstandselement 3 als Heizelement bezogen auf die Draufsicht in etwa in einem mittleren Bereich des Heizblocks 1 angeordnet ist. Ferner sind am Heizblock 1 zwei als Durchgangslöcher ausgebildete Dochtaussparungen 4, 5 vorgesehen, die jeweils gleich mit einem gleichen Abstand zum Widerstandselement 3 für eine symmetrische Anordnung der Dochtaussparungen 4, 5 bezüglich des Widerstandselements 3 ausgebildet sind. Ein Schnitt durch den Heizblock 1 entlang der Linie A-A ist in der Fig. 8 schematisch dargestellt.

Wie dies insbesondere auch aus der Fig. 7 ersichtlich ist, die das Widerstandselement 3 in einer schematischen Darstellung ohne den Heizblock 1 zeigt, ist der stabförmige Widerstandskörper 6, der vorzugsweise aus Keramik mit einem bestimmten Gehalt an Al₂O₃ hergestellt ist, mit einer Widerstandsschicht 7 aus Metalloxid, z. B. einer Nickel-Chromlegierungsschicht, beschichtet. Zur Einstellung eines bestimmten Widerstandswertes ist diese Widerstandsschicht 7 spiralförmig, z. B. durch Laserspiralschneiden, eingeschnitten, so dass ein Spiraleinschnitt um den spiralförmigen, zylindrischen Widerstandskörper 6 herum ausgebildet ist.

Auf die Enden des beschichteten, stabförmigen, zylindrischen Widerstandskörpers 6 ist für eine elektrische Verbindung zu der Widerstandsschicht 7 jeweils eine Metallkappe 8, 9 vorzugsweise aufgepresst. An diesen Kappen 8, 9 ist jeweils eine elektrische Leitung 10, 11, vorzugsweise ein Kupferdraht, angeschweißt, die mit einem Isolationsmaterial isoliert sind.

Über diese elektrischen Leitungen 10, 11 ist das Widerstandselement, wie dies insbesondere aus den Figuren 1 bis 3 ersichtlich ist, mit einem Anschlussstecker 12 verbunden.

Das Widerstandselement 3 ist in eine zentrale Aussparung 13 des Heizblocks 1 einbringbar und wird dort mit einem eine hohe Wärmeleitfähigkeit aufweisenden Material, z. B. einem flammenbeständigen Isolationszement, vergossen, was hier jedoch nicht dargestellt ist. An gegenüberliegenden Aussparungswänden ist zu beiden Seiten des in die Aussparung 13 eingebrachten Widerstandselementes 3 jeweils ein Schlitz 14, 15 ausgebildet, wie dies insbesondere aus den Figuren 1, 3 und 8 ersichtlich ist. Durch diese Schlitze 14, 15 können die elektrischen Leitungen 10, 11 aus dem Heizblock 1 zu dem Anschlussstecker 12 herausgeführt werden.

Wie dies weiter aus den Figuren 4 bis 6 ersichtlich ist, umfasst die Verdampfungsvorrichtung 2 ferner ein Behältnis 16 mit zwei voneinander abgetrennten Kammern 17, 18 als Behälter, wobei in diesen Kammern jeweils unterschiedliche zu verdampfenden Substanzen aufgenommen sind. In diese Kammern 17, 18 ist, wie dies insbesondere aus der Fig. 4 ersichtlich ist, jeweils ein Docht 19, 20 so einsetzbar, dass dieser über einen Dochthalterring 21, 22 an den Kammern 17, 18 festlegbar ist, wobei jeweils eine Dochtende 23, 24 von den Kammern 17, 18 hervorsteht.

Wie dies insbesondere den Figuren 5 und 6 entnommen werden kann, umfasst die Verdampfungsvorrichtung 2 ferner ein aus einer Unterschale 25 und einer Oberschale 26 bestehendes Gehäuse 27 in dem die Heizeinrichtung mitsamt Heizblock 1 aufgenommen ist. Während des Montierens wird das Behältnis 16 so mit der Gehäuse-Unterschale 25 bzw. mit dem Heizblock 1 verbunden, dass die beiden Dochtenden 23, 24 in die Dochtaussparungen 4, 5 am Heizblock 1 hineinragen, was insbesondere aus der Fig. 5 ersichtlich ist. Anschließend wird dann die Gehäuse-Oberschale 26 mit der Gehäuse-Unterschale 25 verclipst, so dass sich die in der Fig. 6 dargestellte fertig montierte Verdampfungsvorrichtung 2 ergibt.

In der Gehäuse-Oberschale 26 ist ferner ein Lüftungsschlitz 28 ausgebildet, über den die verdampften Substanzen in die Umgebung entweichen können.

Beim Betrieb der Verdampfungsvorrichtung 2 wird der Heizblock 1 so erwärmt, dass sich im Bereich der Dochtaussparungen 4, 5 eine Verdampfungstemperatur einstellt, die bewirkt, dass die mittels der Dochte 19, 20 aus den Kammern 17, 18 geförderten Substanzen entsprechend verdampft werden können. Beispielsweise können hier im Rahmen einer Aromatherapie zwei unterschiedliche Duftstoffe in den Kammern 17, 18 aufgenommen sein, die dann beide zusammen mittels der Verdampfungsvorrichtung 2 zur Erzeugung eines Duftstoffgemisches verdampft werden können. Ein entsprechender Einsatz ist auch in Verbindung mit Insektiziden möglich, bei dem z. B. in den beiden Kammern 17, 18 auf unterschiedliche Insekten abgestimmte Insektizide vorhanden sind, die ebenfalls gleichzeitig verdampft werden können. Alternativ dazu kann jedoch auch lediglich ein Behälter mit einem Docht einer der Dochtaussparungen 4, 5 zugeordnet werden, so dass auch der Betrieb dieser Verdampfungsvorrichtung 2 in herkömmlicher Weise mit einem Behälter möglich ist, was hier jedoch nicht dargestellt ist.

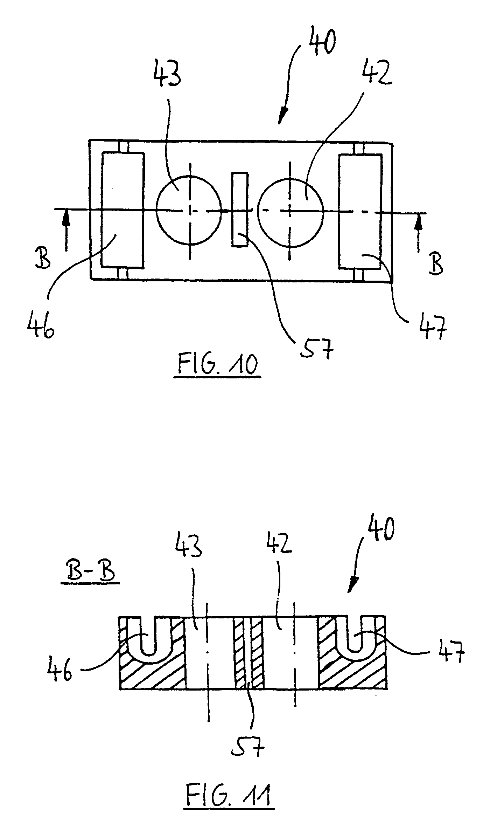

In Fig. 9 ist eine alternative Ausführungsform eines Heizblocks 40 einer Verdampfungsvorrichtung 41 gezeigt, bei der der Heizblock 40 zwei voneinander beabstandete Dochtaussparungen 42, 43 aufweist, die bezogen auf die in der Fig. 10 dargestellte Draufsicht auf den Heizblock 40 in einem mittleren Bereich desselben zwischen zwei im randseitigen Bereich des Heizblocks angeordneten Widerstandselementen 44, 45 (Fig. 9) angeordnet sind. In der Fig. 11 ist ein Querschnitt entlang der Linie B-B durch den Heizblock 40 der Fig. 10 dargestellt.

Die Widerstandselemente 44, 45 entsprechen vom Aufbau her dem Widerstandselement 3, wie es in Verbindung mit den Figuren 1 bis 8 ausführlich beschrieben worden ist, so dass hierauf nicht mehr näher eingegangen wird. Vorzugsweise weisen die beiden Widerstandselemente 44, 45 unterschiedliche Widerstandwerte auf.

Die Widerstandselemente 44, 45 sind in Aussparungen 46, 47 am Heizblock 40 aufgenommen und dort einzementiert, wobei jedem Widerstandselement 44, 45 elektrische Anschlussleitungen 48, 49 sowie 50, 51 zugeordnet sind, die durch Schlitze im Bereich der Aussparungsseitenwände herausgeführt sind dergestalt, dass die Anschlussleitung 48 des Widerstandselements 44 sowie die Anschlussleitung 49 des Widerstandselements 45 jeweils zu einem Handschalter 52 geführt sind. Dagegen sind die Anschlussleitung 50 des Widerstandselements 44 und die Anschlussleitung 51 des Widerstandselements 45 zu einem Anschlussstecker 53 geführt. Weiter ist eine elektrische Leitung 54 vom Handschalter 52 zum Anschlussstecker 53 geführt.

Wie dies den Figuren 9 bis 11 ferner entnommen werden kann, ist zur zumindest teilweisen thermischen Entkopplung der beiden durch die Dochtaussparung 42 und das Widerstandselement 44 sowie die Dochtaussparung 43 und das Widerstandselement 45 gebildeten Heizeinheiten 55 bzw. 56 in einem Bereich zwischen den beiden Dochtaussparungen 42, 43 ein durch den Heizblock 40 durchgehender Luftspalt 57 als Trennmittel vorgesehen.

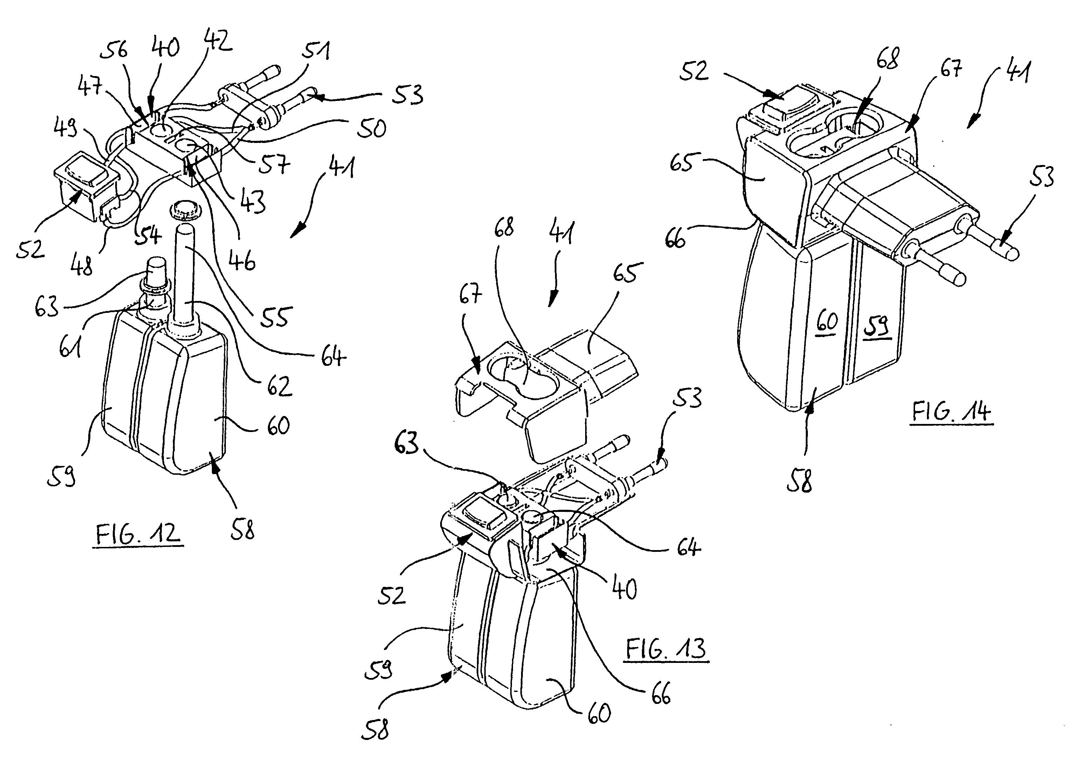

Wie dies den Figuren 12 bis 14 entnommen werden kann, weist die Verdampfungsvorrichtung 41 ferner ein Behältnis 58 mit zwei Kammern 59, 60 als Behälter auf, wobei jeder Kammer jeweils ein Docht 61, 62 zugeordnet ist, der im montierten Zustand mit einem Dochtende 63, 64 aus den beiden Kammern 59, 60 hervorsteht. In den beiden Kammern (59, 60) sind vorzugsweise unterschiedliche zu verdampfende Substanzen enthalten, die eine unterschiedliche Verdampfungstemperatur aufweisen.

Im montierten Zustand ist der Heizblock 40 in einem Gehäuse 67, das wiederum aus einer Gehäuse-Oberschale 65 und einer Gehäuse-Unterschale 66 aufgebaut ist, aufgenommen, wie dies insbesondere aus der Fig. 13 ersichtlich ist. An diesem Gehäuse 67 kann ferner eine Aussparung zur Integration des Handschalters 52 vorgesehen sein. Weiter umfasst die Oberschale 65 einen Lüftungsschlitz 68.

Über den Handschalter 52 können die beiden Heizeinheiten 55, 56 einzeln aktiviert werden, so dass entweder die in der Kammer 59 oder die in der Kammer 60 aufgenommene flüchtige Substanz verdampft wird. Die Widerstandselemente 44, 45 können dabei in ihrem Widerstandswert entsprechend an die zu verdampfenden Substanzen in den Kammern 59, 60 angepasst sein. Der Handschalter 52 kann zudem noch eine weitere Schaltposition aufweisen, mit der beide Heizeinheiten 55, 56 aktiviert sind, so dass, insbesondere im Rahmen einer Aromatherapie ein Duftstoffgemisch durch Verdampfen aus beiden Kammern 59, 60 erzeugt werden kann. In einer weiteren Schaltposition des Handschalters 52 sind dagegen beide Heizeinheiten 55, 56 deaktiviert. Diese Schaltstellungen des Handschalters 52 sind hier nicht im Detail dargestellt.

## Patentansprüche

1. Vorrichtung zum Verdampfen von flüchtigen Substanzen, insbesondere von Insektiziden und/oder Duftstoffen,
mit einem Gehäuse (27; 67),
mit einer im Gehäuse (27; 67) angeordneten Heizeinrichtung, die einen Heizblock (1; 40), vorzugsweise aus Keramik, umfasst, der zur Erwärmung des Heizblocks (1; 40) ein Heizelement (3; 44) aufweist, und
mit einem mit dem Gehäuse (27; 67) verbindbaren Behälter (17; 59) für eine zu verdampfende Substanz, wobei in den Behälter (17; 59) ein Docht (19; 61) einsetzbar ist, der bei mit dem Gehäuse (27; 67) verbundenem Behälter (17; 59) zur Verdampfung der sich im Behälter (17; 59) befindlichen Substanz mit einem aus dem Behälter (17; 59) ragenden Dochtende (23; 63) in eine Dochtaussparung (4; 42) des Heizblocks (1; 40) ragt,
**dadurch gekennzeichnet,**
**dass** im Heizblock (1; 40) wenigstens eine weitere Dochtaussparung (5; 43) ausgebildet ist, und
**dass** jeder weiteren Dochtaussparung (5; 43) jeweils ein weiterer Behälter (18; 60) mit darin einsetzbarem Docht (20; 62) so zugeordnet ist, dass ein Dochtende (24; 64) des Dochtes (20; 62) des weiteren Behälters (18; 60) zur Verdampfung der sich in diesem weiteren Behälter (18; 60) befindlichen Substanz in die weitere Dochtaussparung (5; 43) hineinragt.

2. Vorrichtung nach Anspruch 1, **dadurch gekennzeichnet,**
**dass** das Gehäuse (1; 40) mit zwei Behältern (17, 18; 59, 60) verbindbar ist, die jeweils einen Docht (19, 20; 61, 62) aufweisen, und
**dass** im Heizblock (1; 40) zwei Dochtaussparungen (4, 5; 42, 43) ausgebildet sind, in die jeweils ein Dochtende (23, 24; 63, 64) eines jedem Behälter (17, 18; 59, 60) zugeordneten Dochtes (19, 20; 61, 62) ragt.

3. Vorrichtung nach Anspruch 2, **dadurch gekennzeichnet, dass** die beiden Behälter jeweils separate Behältnisse sind oder durch ein einziges Behältnis (16; 58) mit zwei voneinander abgetrennten Kammern als Behälter (17, 18; 59, 60) gebildet sind, wobei in den beiden Behältern (17, 18; 59, 60) vorzugsweise unterschiedliche zu verdampfende Substanzen aufgenommen sind.

4. Vorrichtung nach einem der Ansprüche 1 bis 3, **dadurch gekennzeichnet, dass** das Heizelement durch ein elektrisches Widerstandselement (3; 44, 45) gebildet ist, das im Heizblock (1; 40) aufgenommen ist.

5. Vorrichtung nach einem der Ansprüche 1 bis 4, **dadurch gekennzeichnet, dass** die Dochtaussparungen (4, 5; 42, 43) als Durchgangsloch oder als am Heizblock (1; 40) randseitig durchgehende Einbuchtung ausgebildet sind.

6. Vorrichtung nach einem der Ansprüche 2 bis 5, **dadurch gekennzeichnet,**
**dass** der Heizblock (1) in einer Draufsicht eine rechteckige oder in etwa ovale Form aufweist,
**dass** das Heizelement (3) bezogen auf die Draufsicht in etwa in einem mittleren Bereich des Heizblocks (1) angeordnet ist, und
**dass** bezogen auf die Draufsicht jeweils eine Dochtaussparung (4, 5) zu beiden Seiten des Heizelements (3) ausgebildet ist.

7. Vorrichtung nach einem der Ansprüche 2 bis 6, **dadurch gekennzeichnet, dass** die Dochtaussparungen (4, 5) jeweils gleich mit einem gleichen Abstand zum Heizelement (3) für eine vorzugsweise symmetrische Anordnung der Dochtaussparungen (4, 5) bezüglich des Heizelements (3) ausgebildet sind.

8. Vorrichtung nach einem der Ansprüche 1 bis 5, **dadurch gekennzeichnet,**
**dass** wenigstens zwei Heizelemente (44, 45) am Heizblock (40) vorgesehen sind, wobei jedes Heizelement (44, 45) wenigstens einer Dochtaussparung (42, 43) zugeordnet ist und wobei jede Dochtaussparung (42, 43) sowie jedes einer Dochtaussparung (42, 43) zugeordnete Heizelement (44, 45) jeweils eine Heizeinheit (55, 56) bilden, und
**dass** die Heizelemente (44, 45) mit einer Schalt- und/oder Steuereinrichtung (52) gekoppelt sind, über die die Heizelemente (44, 45) zusammen deaktivierbar sowie ggf. aktivierbar sind und über die die Heizelemente (44, 45) einzeln aktivierbar und deaktivierbar sind.

9. Vorrichtung nach Anspruch 8, **dadurch gekennzeichnet, dass** zwei Heizelemente (44, 45) vorgesehen sind, die jeweils durch ein elektrisches Widerstandselement gebildet sind und die beabstandet voneinander am Heizblock (40) angeordnet sind.

10. Vorrichtung nach Anspruch 9, **dadurch gekennzeichnet, dass** die elektrischen Widerstandselemente (44, 45) in etwa stabförmig ausgebildet sind und in etwa parallel zueinander ausgerichtet sind.

11. Vorrichtung nach Anspruch 9 oder Anspruch 10, **dadurch gekennzeichnet, dass** zwei Dochtaussparungen (42, 43) beabstandet voneinander sowie bezogen auf eine Draufsicht auf den Heizblock (40) in einem mittleren Bereich zwischen zwei vorzugsweise im randseitigen Bereich des Heizblocks (40) angeordneten Heizelementen (44, 45) angeordnet sind.

12. Vorrichtung nach Anspruch 11, **dadurch gekennzeichnet, dass** zur zumindest teilweisen thermischen Entkopplung der beiden durch je ein Heizelement (44, 45) und eine zugeordnete Dochtaussparung (42, 43) gebildeten Heizeinheiten (55, 56) in einem Bereich zwischen den beiden Dochtaussparungen (42, 43) wenigstens ein Trennmittel (57) vorgesehen ist.

13. Vorrichtung nach Anspruch 12, **dadurch gekennzeichnet, dass** das Trennmittel zur thermischen Entkopplung durch einen wenigstens im Bereich zwischen den beiden Dochtaussparungen (42, 43) durch den Heizblock (40) durchgehenden Luftspalt (57) gebildet ist.

14. Vorrichtung nach einem der Ansprüche 8 bis 13, **dadurch gekennzeichnet, dass** die Heizelemente (44, 45) eine unterschiedliche Heizleistung bereitstellen, insbesondere im Falle eines elektrischen Widerstandselementes unterschiedliche vorgebbare Widerstandswerte aufweisen.

15. Vorrichtung nach einem der Ansprüche 8 bis 14, **dadurch gekennzeichnet, dass** die Schalt- und/oder Steuereinrichtung durch einen Handschalter (52) oder einen programmierbaren Mikroprozessor gebildet ist.

16. Vorrichtung nach einem der Ansprüche 1 bis 15, **dadurch gekennzeichnet, dass** jedes Heizelement (3; 44, 45) über elektrische Leitungen (10, 11; 48, 49, 50, 51) mit einem am Gehäuse (1; 40) angeordneten Anschlussstecker (12; 53) gekoppelt ist.

17. Vorrichtung nach einem der Ansprüche 4 bis 16, **dadurch gekennzeichnet, dass** jedes elektrische Widerstandselement (3; 44, 45) zur Ausbildung einer Heizeinrichtung mit geringen Abmessungen und damit einer insgesamt miniaturisierten Vorrichtung (2; 41) zum Verdampfen von flüchtigen Substanzen einen stabförmigen Widerstandskörper (6) aufweist, der wenigstens bereichsweise mit einer Widerstandsschicht (7) beschichtet ist, die zur Einstellung eines bestimmten Widerstandswertes entsprechend einer auf die Zusammensetzung der jeweils zu verdampfenden Substanz abgestimmten Verdampfungstemperatur bereichsweise eingeschnitten und/oder bereichsweise eingeschliffen ist.

18. Vorrichtung nach Anspruch 17, **dadurch gekennzeichnet, dass** die Widerstandsschicht (7) um den stabförmigen, vorzugsweise zylinderförmigen Widerstandskörper (6) herum spiralförmig mit einem Spiralschnitt eingeschnitten ist, vorzugsweise durch Laserspiralschneiden.

19. Vorrichtung nach Anspruch 17 oder Anspruch 18, **dadurch gekennzeichnet,**
**dass** das stabförmige Widerstandselement (3; 44, 45) in eine Aussparung (13; 46, 47) des Heizblocks (1; 40) einbringbar ist und dort mit einem eine hohe Wärmeleitfähigkeit aufweisenden Material, vorzugsweise einem flammenbeständigen Isolationszement, vergossen ist, und
**dass** an gegenüberliegenden Aussparungswänden zu beiden Seiten des Widerstandselementes (3; 44, 45) jeweils ein Schlitz (14, 15)ausgebildet ist, durch die die elektrischen Leitungen (10, 11; 48, 49, 50, 51) aus dem Heizblock (1; 40) zu einem Anschlussstecker (12; 53) hin herausgeführt sind.

20. Vorrichtung nach einem der vorhergehenden Ansprüche, **dadurch gekennzeichnet,**
**dass** das Gehäuse (27; 67) wenigstens zweiteilig aus einer Oberschale (26; 65) und einer Unterschale (25; 66) aufgebaut ist, die vorzugsweise miteinander durch Rast- und/oder Clipelemente verbindbar sind,
**dass** die Unterschale (25; 66) Verbindungsmittel zur Verbindung des Behälters mit dem Gehäuse (27; 67) aufweist, und
**dass** wenigstens eine der beiden Schalen (25, 26, 65, 66), vorzugsweise die Oberschale (26; 65) im Bereich oberhalb der Dochtenden 23, 24; 63, 64), wenigstens einen Lüftungsschlitz (28; 68) zum Entweichen der verdampften Substanzen aufweist.

## Claims

1. Device for vaporizing volatile substances, in particular insecticides and/or perfumes,
with a housing (27; 67),
with a heating arrangement which is disposed in the housing (27; 67) and which comprises a heating block (1; 40), preferably of ceramic, with a heating element (3; 44) for warming the heating block (1; 40), and
with a container (17; 59) for a substance to be vaporized, which container (17; 59) can be connected to the housing (27; 67) and into which container (17; 59) it is possible to insert a wick (19; 61) which, when the container (17; 59) is connected to the housing (27; 67), has a wick end (23; 63) protruding from the container (17; 59) into a wick recess (4; 42) of the heating block (1; 40) in order to vaporize the substance located in the container (17; 59),
**characterized in that**
at least one further wick recess (5; 43) is formed in the heating block (1; 40), and
each further wick recess (5; 43) is assigned in each case a further container (18; 60) with a wick (20; 62) insertable therein, so that a wick end (24; 64) of the wick (20; 62) of the further container (18; 60) protrudes into the further wick recess (5; 43) in order to vaporize the substance located in this further container (18; 60).

2. Device according to Claim 1, **characterized in that**
the housing (1; 40) can be connected to two containers (17, 18; 59, 60) which each have a wick (19, 20; 61, 62), and
two wick recesses (4, 5; 42, 43) are formed in the heating block (1; 40), into each of which wick recesses (4, 5; 42, 43) a wick end (23, 24; 63, 64) of a wick (19, 20; 61, 62) assigned to each container (17, 18; 59, 60) protrudes.

3. Device according to Claim 2, **characterized in that** the two containers are each separate receptacles or are formed by a single receptacle (16; 58) with two chambers separate from one another as containers (17, 18; 59, 60), the two containers (17, 18; 59, 60) preferably receiving different substances to be vaporized.

4. Device according to one of Claims 1 to 3, **characterized in that** the heating element is formed by an electrical resistance element (3; 44, 45) accommodated in the heating block (1; 40).

5. Device according to one of Claims 1 to 4, **characterized in that** the wick recesses (4, 5; 42, 43) are designed as a through-hole or as a continuous indentation made at the edge of the heating block (1; 40).

6. Device according to one of Claims 2 to 5, **characterized in that**, in a plan view, the heating block (1) has a rectangular or approximately oval shape,
the heating element (3), in relation to the plan view, is arranged approximately in a central area of the heating block (1), and,
in relation to the plan view, a respective wick recess (4, 5) is formed to both sides of the heating element (3).

7. Device according to one of Claims 2 to 6, **characterized in that** the wick recesses (4, 5) are each identically designed equidistant from the heating element (3) for a preferably symmetrical arrangement of the wick recesses (4, 5) relative to the heating element (3).

8. Device according to one of Claims 1 to 5, **characterized in that**
at least two heating elements (44, 45) are provided on the heating block (40), each heating element (44, 45) being assigned to at least one wick recess (42, 43), and each wick recess (42, 43) and each heating element (44, 45) assigned to a wick recess (42, 43) form a heating unit (55, 56), and
the heating elements (44, 45) are coupled to a switching and/or controlling means (52) via which the heating elements (44, 45) can be jointly deactivated and activated, and via which the heating elements (44, 45) can be individually activated and deactivated.

9. Device according to Claim 8, **characterized in that** two heating elements (44, 45) are provided which are each formed by an electrical resistance element and are spaced apart from one another on the heating block (40).

10. Device according to Claim 9, **characterized in that** the electrical resistance elements (44, 45) are of approximately rod-shaped design and are oriented approximately parallel to one another.

11. Device according to Claim 9 or Claim 10, **characterized in that** two wick recesses (42, 43) are arranged spaced apart from one another and, in relation to a plan view of the heating block (40), are arranged in a central area between two heating elements (44, 45) preferably arranged in the edge area of the heating block (40).

12. Device according to Claim 11, **characterized in that**, in order to permit at least partial thermal uncoupling of the two heating units (55, 56) formed in each case by a heating element (44, 45) and an associated wick recess (42, 43), at least one separating means (57) is provided in an area between the two wick recesses (42, 43).

13. Device according to Claim 12, **characterized in that** the separating means for thermal uncoupling is formed by an air gap (57) extending through the heating block (40) at least in the area between the two wick recesses (42, 43).

14. Device according to one of Claims 8 to 13, **characterized in that** the heating elements (44, 45) provide a different heat output, having, particularly in the case of an electrical resistance element, different predeterminable resistance values.

15. Device according to one of Claims 8 to 14, **characterized in that** the switching and/or controlling means is formed by a manual switch (52) or a programmable microprocessor.

16. Device according to one of Claims 1 to 15, **characterized in that** each heating element (3; 44, 45) is coupled via electrical lines (10, 11; 48, 49, 50, 51) to a connector plug (12; 53) arranged on the housing (1; 40).

17. Device according to one of Claims 4 to 16, **characterized in that**, in order to form a heating arrangement with small dimensions and thus an overall miniaturized device (2; 41) for vaporizing volatile substances, each electrical resistance element (3; 44, 45) has a rod-shaped resistance body (6) which is coated at least in some areas with a resistance layer (7) which, in order to set a defined resistance value corresponding to a vaporization temperature for the composition of the respective substance to be vaporized, is notched in some areas and/or ground-in in some areas.

18. Device according to Claim 17, **characterized in that** the resistance layer (7) around the rod-shaped, preferably cylinder-shaped resistance body (6), is notched in a helical formation with a helical cut, preferably by laser helical cutting.

19. Device according to Claim 17 or Claim 18, **characterized in that**
the rod-shaped resistance element (3; 44, 45) can be introduced into a recess (13; 46, 47) of the heating block (1; 40) and can there be encapsulated with a material having high thermal conductivity, preferably a flame-resistant insulation cement, and,
at opposite recess walls on both sides of the resistance element (3; 44, 45) a slit (14, 15) is in each case formed through which the electrical lines (10, 11; 48, 49, 50, 51) are routed from the heating block (1; 40) to a connector plug (12; 53).

20. Device according to one of the preceding claims, **characterized in that**
the housing (27; 67) is made up of at least two parts, namely an upper shell (26; 65) and a lower shell (25; 66), which can preferably be connected to one another by catch elements and/or clip elements,
the lower shell (25; 66) has connector means for connecting the container to the housing (27; 67), and
at least one of the two shells (25, 26; 65, 66), preferably the upper shell (26; 65), has, in the area above the wick ends (23, 24; 63, 64), at least one ventilation slot (28; 68) for escape of the vaporized substances.

## Revendications

1. Dispositif de vaporisation de substances liquides, notamment insecticides et/ou parfums,
avec un boîtier (27; 67),
avec un dispositif de chauffage disposé dans le boîtier (27; 67) qui comprend un bloc chauffant (1; 40), de préférence en céramique, qui comprend un élément chauffant (3; 44) pour réchauffer le bloc chauffant (1; 40), et
avec un récipient (17; 59) connectable au boîtier (27; 67) pour une substance à vaporiser, sachant qu'une mèche (19; 61) peut être insérée dans le récipient (17; 59), laquelle mèche, avec le récipient (17; 59) connecté au boîtier (27, 67), fait saillie dans un évidement pour mèche (4; 42) du bloc chauffant (1; 40) par une extrémité de mèche (23; 63) faisant saillie hors du récipient (17; 59) pour la vaporisation de la substance se trouvant dans le récipient (17; 59),
**caractérisé en ce que**
au moins un autre évidement pour mèche (5; 43) est formé dans le bloc chauffant (1; 40), et
**en ce qu'**un autre récipient (18; 60) avec une mèche (20; 62) insérable dedans est respectivement associé à chaque autre évidement pour mèche (5; 43) de telle manière qu'une extrémité de mèche (24; 64) de la mèche (20; 62) de l'autre récipient (18; 60) fait saillie dans l'autre évidement pour mèche (5; 43) pour la vaporisation de la substance se trouvant dans cet autre récipient (18; 60).

2. Dispositif selon la revendication 1, **caractérisé en ce que**
le boîtier (1; 40) est connectable à deux récipients (17, 18; 59, 60) qui comportent chacun une mèche (19, 20; 61, 62), et **en ce que** deux évidements pour mèche (4, 5; 42, 43) sont formés dans le bloc chauffant (1; 40) dans lesquels une extrémité de mèche (23, 24; 63, 64) d'une mèche (19, 20; 61, 62) associée à chaque récipient (17, 18; 59, 60) fait saillie.

3. Dispositif selon la revendication 2, **caractérisé en ce que** les deux récipients sont des récipients séparés ou sont formés par un seul récipient (16; 58) avec deux chambres séparées l'une de l'autre comme des récipient (17, 18; 59, 60), moyennant quoi les deux récipients (17, 18; 59, 60) contiennent, de préférence, des substances à vaporiser différentes.

4. Dispositif selon l'une quelconque des revendications 1 à 3, **caractérisé en ce que** l'élément chauffant est formé par un élément de résistance électrique (3; 44, 45) qui est logé dans le bloc chauffant (1; 40).

5. Dispositif selon l'une quelconque des revendications 1 à 4, **caractérisé en ce que** les évidements pour mèche (4, 5; 42, 43) sont configurés comme trou passant ou comme indentation continue du côté du bord du bloc chauffant (1 ; 40).

6. Dispositif selon l'une quelconque des revendications 2 à 5, **caractérisé en ce que** le bloc chauffant (1) présente une forme rectangulaire ou à peu près ovale en vue de dessus,
**en ce que** l'élément chauffant (3) est disposé à peu près dans une zone médiane du bloc chauffant (1) par rapport à la vue de dessus, et
**en ce qu'**un évidement pour mèche (4, 5) est formé respectivement de chaque côté de l'élément chauffant (3) par rapport à la vue de dessus.

7. Dispositif selon l'une quelconque des revendications 2 à 6, **caractérisé en ce que** les évidements pour mèche (4, 5) sont configurés respectivement identiques avec une distance identique par rapport à l'élément chauffant (3) pour une disposition de préférence symétrique des évidements pour mèche (4, 5) par rapport à l'élément chauffant (3).

8. Dispositif selon l'une quelconque des revendications 1 à 5, **caractérisé en ce que**
au moins deux éléments chauffants (44, 45) sont prévus sur le bloc chauffant (40), moyennant quoi chaque élément chauffant (44, 45) est associé à au moins un évidement pour mèche (42, 43) et chaque évidement pour mèche (42, 43) ainsi que chaque élément chauffant (44, 45) associé à un évidement pour mèche (42, 43) forment respectivement une unité chauffante (55, 56), et
les éléments chauffants (44, 45) sont couplés avec un dispositif de commutation et/ou de commande (52) par l'intermédiaire duquel les éléments chauffants (44, 45) peuvent être désactivés, et éventuellement activés, ensemble et par l'intermédiaire duquel les éléments chauffants (44, 45) peuvent être activés et désactivés individuellement.

9. Dispositif selon la revendication 8, **caractérisé en ce qu'**il est prévu deux éléments chauffants (44, 45) qui sont formés chacun par un élément de résistance électrique et qui disposés sur le bloc chauffant (40) à distance l'un de l'autre.

10. Dispositif selon la revendication 9, **caractérisé en ce que** les éléments de résistance électrique (44, 45) sont à peu près en forme de barre et sont orientés à peu près parallèlement l'un à l'autre.

11. Dispositif selon la revendication 9 ou la revendication 10, **caractérisé en ce que** deux évidements pour mèche (42, 43) sont disposés à distance l'un de l'autre et, par rapport à une vue de dessus du bloc chauffant (40), dans une zone médiane entre deux éléments chauffants (44, 45) disposés, de préférence, dans la zone du côté du bord du bloc chauffant (40).

12. Dispositif selon la revendication 11, **caractérisé en ce que**, au moins pour le découplage thermique partiel des deux unités chauffantes (55, 56) formées respectivement par un élément chauffant (44, 45) et un évidement pour mèche (42, 43) associé, au moins un moyen de séparation (57) est prévu dans une zone entre les deux évidements pour mèche (42, 43).

13. Dispositif selon la revendication 12, **caractérisé en ce que** le moyen de séparation pour le découplage thermique est formé par un entrefer (57) passant à travers le bloc chauffant (40) au moins dans la zone entre les deux évidements pour mèche (42, 43).

14. Dispositif selon l'une quelconque des revendications 8 à 13, **caractérisé en ce que** les éléments chauffants (44, 45) fournissent une puissance de chauffage variable, en particulier dans le cas d'un élément de résistance électrique, qu'ils présentent des valeurs de résistance variables prédéterminables.

15. Dispositif selon l'une quelconque des revendications 8 à 14, **caractérisé en ce que** le dispositif de commutation et/ou de commande est formé par un commutateur manuel (52) ou un microprocesseur programmable.

16. Dispositif selon l'une quelconque des revendications 1 à 15, **caractérisé en ce que** chaque élément chauffant (3; 44, 45) est couplé par des fils électriques (10, 11; 48, 49, 50, 51) à un connecteur (12; 53) disposé dans le boîtier (1; 40).

17. Dispositif selon l'une quelconque des revendications 4 à 16, **caractérisé en ce que** chaque élément de résistance électrique (3; 44, 45) comporte, pour la configuration d'un dispositif chauffant avec des dimensions réduites et, donc, d'un dispositif miniaturisé (2; 41) pour la vaporisation de substances liquides, un corps de résistance en forme de barre (6) qui est revêtu au moins par zones d'une couche résistive (7) qui est entaillée par zones et/ou meulée par zones pour le réglage d'une certaine valeur de résistance en fonction d'une température de vaporisation adaptée à la composition de la substance respective à vaporiser.

18. Dispositif selon la revendications 17, **caractérisé en ce que** la couche résistive (7) est entaillée en forme de spirale avec une entaille hélicoïdale autour du corps de résistance en forme de barre (6), de préférence en forme de cylindre, de préférence par découpage hélicoïdal au laser.

19. Dispositif selon la revendications 17 ou la revendication 18, **caractérisé en ce que**
l'élément de résistance en forme de barre (3; 44, 45) peut être placé dans un évidement (13; 46, 47) du bloc chauffant (1; 40) et **en ce qu'**il est coulé avec une matière ayant une conductibilité thermique élevée, de préférence un ciment isolant résistant aux flammes, et
**en ce que**, dans des parois d'évidements opposées des deux côtés de l'élément de résistance (3; 44, 45), une fente (14, 15) est respectivement formée à travers laquelle les fils électriques (10, 11; 48, 49, 50, 51) sont amenés à partir du bloc chauffant 1; 40) vers le connecteur (12; 53).

20. Dispositif selon l'une quelconque des revendications précédentes, **caractérisé en ce que**
le boîtier (27; 67) est constitué au moins de deux parties, une coque supérieure (26 ; 65) et une coque inférieure (25; 66), qui sont, de préférence, connectables l'une avec l'autre à l'aide d'éléments à crans et/ou à clips,
la coque inférieure (25; 66) comporte des moyens de connexion pour la connexion du récipient avec le boîtier (27; 67), et
au moins l'une des deux coques (25, 26; 65, 66), de préférence la coque supérieure (26; 65), comporte, dans la zone au-dessus des extrémités de mèche (23, 24; 63, 64), au moins une fente d'aération (28; 68) pour l'échappement des substances vaporisées.
